# EUROPEAN PATENT SPECIFICATION

(11) **EP 3 194 610 B1**
(45) Date of publication and mention of the grant of the patent: **13.11.2019**
(21) Application number: 15763330.6
(22) Date of filing: 15.09.2015
(51) Int. Cl.: C12Q 1/37, G01N 33/569, G01N 33/573, C07K 16/00, C07K 16/28

(54) **ANTI LI P10 ANTIBODY**
ANTI LI P10 ANTIKÖRPER
ANTICORPS ANTI LI P10

(30) Priority: 18.09.2014 EP 14185409
(43) Date of publication of application: 26.07.2017
(73) Proprietor: F. Hoffmann-La Roche AG, 4070 Basel (CH)
(72) Inventor: BENTO PEREIRA DA SILVA, Ana Patricia, 4056 Basel (CH); DZIADEK, Sebastian, 83671 Benediktbeuern (DE); ECABERT, Barbara, 4106 Therwil (CH); GERG, Michael, 81377 Muenchen (DE); NOGOCEKE, Everson, 4052 Basel (CH); MARCINOWSKI, Moritz, 68165 Mannheim (DE); REIS, Bernhard, 4053 Basel (CH); SCHINDLER, Thomas, 79540 Loerrach (DE); THERON, Michel Achille Edouard, 68270 Ruelisheim (FR)
(74) Representative: Küng, Peter
(86) International application number: PCT/EP2015/071002
(87) International publication number: WO 2016/041915

(56) References cited:
- WO-A1-02/21129
- WO-A1-2012/041824
- WO-A1-2012/104344

## Description

The present invention provides an antibody directed to li p10 peptide. It can be used in methods for monitoring Cathepsin S inhibition.

Cathepsin S is well known for its critical function in the proteolytic digestion of the invariant chain chaperone molecules, thus controlling antigen presentation to CD4⁺ T-cells by major histocompatibility complex (MHC) class II molecules or to NK1.1+ T-cells via CD1 molecules. Cathepsin S also appears to participate in direct processing of exogenous antigens for presentation by MHC class II to CD4⁺ T-cells, or in cross-presentation by MHC class I molecules to CD8⁺ T-cells. In addition, in its secreted form Cathepsin S is implicated in degradation of the extracellular matrix, which may contribute to the pathology of a number of diseases, including arthritis, atherosclerosis and chronic obstructive pulmonary disease. Therefore, inhibition of Cathepsin S is a promising target for the development of novel therapeutics for a variety of indications.

MHC class II molecules bind and display antigenic peptides as class II-peptide complexes on the cell surface of antigen presenting cells (APCs) for recognition by CD4⁺ helper cells. The molecular mechanisms leading to formation of class II-peptide complexes and the presentation of antigens on the cell surface begin with synthesis of class II αβ heterodimers in the endoplas-matic reticulum. During biosynthesis these αβ heterodimers associate with a type II membrane protein, the isoforms of the invariant chain li, to form a nonameric complex of αβli. The αβli complex traverses the Golgi apparatus and is delivered to intracellular compartments. In these acidic compartments, the li is degraded and finally liberated from the αβli complexes, allowing class II molecules to bind antigenic peptides. The proteolytical processing of li results in shorter fragments. The cysteine protease Cathepsin S plays a key role in processing li in APCs. Cathepsin S degrades the invariant chain li from class II-li complexes generating class II-CLIP. Hence, inhibition of Cathepsin S leads to a decrease of CLIP peptide and to accumulation of the precursor fragment, i.e. the invariant chain li degradation peptide p10 (li p10).

EP 1 315 966 discloses a method for monitoring the effect of in vivo administration of cathepsin S inhibitors. The method includes the purification of white blood cells from a blood sample of a subject treated with a Cathepsin S inhibitor, preparation of whole cell lysates of the purified white blood cells and analyzing the whole cell lysates for presence of li p10 fragment of invariant chain li. This method requires blood samples of about 10 - 30 ml and is labour inten-sive (isolation of white blood cells and preparation of whole cell lysates). The detection of li p10 is done by ELISA or Western blot.

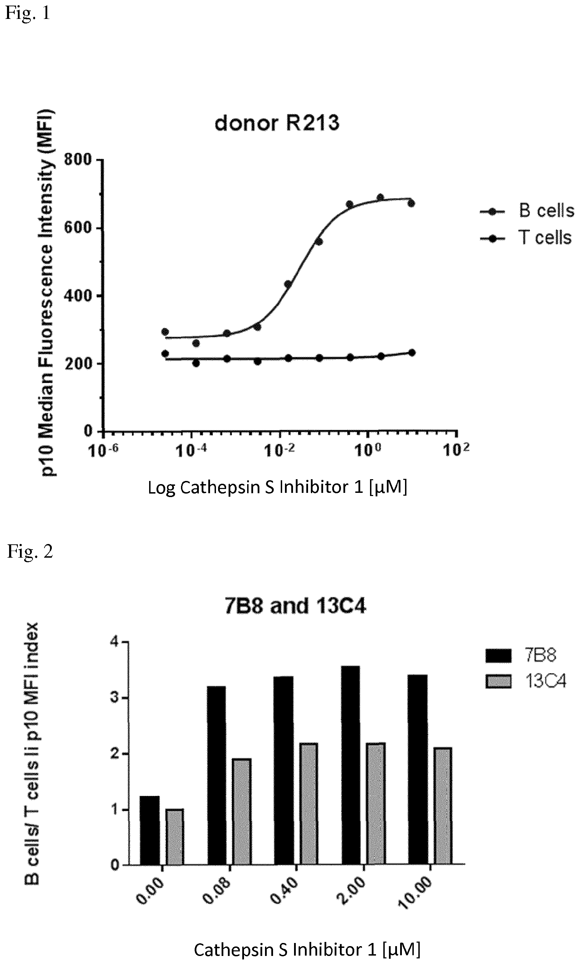

WO 2012/104344 discloses isolated human monoclonal antibodies which bind to human CD74, which is the cell surface form of the invariant chain li. Related antibody-drug conjugates (ADCs) are also disclosed, as well as pharmaceutical compositions comprising the antibodies or antibody-drug conjugates, and therapeutic and diagnostic methods for using the antibodies and/or ADCs. Specific antibodies are disclosed, see the details thereof in Table 3 on p.68, claims 1, 2 and 10-13, as well as Fig. 2.

Disclosed herein is an assay to monitor Cathepsin S inhibition which overcomes at least some of the drawbacks of the prior art methods.

In a first object, it is disclosed a method for monitoring Cathepsin S inhibitor activity in tissue samples of animals comprising:
a) providing a tissue sample of an animal to whom a Cathepsin S inhibitor has been administered or providing a tissue sample of an animal that was contacted *in vitro* with a Cathepsin S inhibitor, wherein the tissue sample comprises white blood cells,
b) measuring li p10 peptide level in white blood cells of the tissue sample of step a) by flow cytometry and
c) correlating li p10 peptide level in white blood cells to Cathepsin S inhibitor dose, wherein a Cathepsin S inhibitor leads to increased level of li p10 peptide in white blood cells.

In a second object, it is disclosed a method for the identification of a Cathepsin S inhibitor comprising:
a) treating a non-human animal with a test compound,
b) providing a tissue sample of the animal of step a) comprising white blood cells,
c) measuring li p10 peptide level in the white blood cells of the tissue sample of step b),
wherein an increased level of li p10 peptide in white blood cells in the sample of step a) compared to a level of li p10 peptide in white blood cells in a tissue sample of an untreated animal is indicative for a Cathepsin S inhibitor.

In a preferred embodiment of said methods, the tissue sample is blood.

In a further preferred embodiment of said methods, the white blood cells are Antigen Presenting Cells (APCs), preferably the APCs are selected from B cells, monocytes, macrophages and dendritic cells, more preferably selected from B cells and monocytes.

The term Antigen Presenting cells (APCs) as used herein refers to cells that display antigenic peptides as (MHC) class II - antigenic peptide complex on its surface. APCs comprise dendritic cells, monocytes, macrophages and B cells.

The invention relates to the third object, that provides an anti-li p10 antibody comprising:
a VH domain comprising a CDR1 comprising the amino acid sequence of Seq. Id. No. 4, a CDR2 comprising the amino acid sequence of Seq. Id. No. 5, CDR3 sequence comprising the amino acid sequence of Seq. Id. No. 6., and a VL domain comprising a CDR1 comprising the amino acid sequence of Seq. Id. No. 7, a CDR2 comprising the amino acid sequence of Seq. Id. No. 8, a CDR3 sequence comprising the amino acid sequence of Seq. Id. No. 9.

In a particular embodiment of the present invention, the VH domain of the anti-li p10 antibody comprises the amino acid sequence of Seq. Id. No. 10 and the VL domain of the anti-li p10 antibody comprises the amino acid sequence of Seq. Id. No. 11.

The anti-li p10 specific antibody is referred to herein as 7B8 neoepitope antibody originating from Oryctolagus cuniculus. This antibody is characterized by CDRs and VH, VL sequences disclosed in Seq. Id. Nos. 4 - 11.

The term Cathepsin S refers to a cysteine protease in the papain super-family. The amino acid sequence of human Cathepsin S is given in Seq. Id. No. 1.

The term "invariant chain li" refers to invariant polypeptide of major histocompatibility complex or CD74. The amino acid sequence of human invariant chain li is given in Seq. Id. No. 2.

The term li p10 peptide refers to class II-associated li peptide. The amino acid sequence of human li p10 is given in Seq. Id. No. 3.

The term "MHC II polypeptide" as used herein refers to the alpha chain and the beta chain constituting the MHC II complex.

The term animal as used herein comprises human beings and non-human animals such as e.g. monkey, dog, pig, rabbit, guinea pig and rodents.

The term "compound" is used herein in the context of a "test compound" or a "drug candidate compound" described in connection with the assays of the present invention. As such, these compounds comprise organic or inorganic compounds, derived synthetically or from natural sources. The compounds include inorganic or organic compounds such as polynucleotides, lipids or hormone analogs that are characterized by relatively low molecular weights. Other biopolymeric organic test compounds include peptides comprising from about 2 to about 40 amino acids and larger polypeptides comprising from about 40 to about 500 amino acids, such as antibodies or antibody conjugates.

The inventors have surprisingly found that li p10 peptide can be detected in white blood cells. The methods disclosed herein have the advantage compared to the methods described in the prior art that tissue samples, preferably whole blood, can be assayed directly without involving the isolation of white blood cells. In the methods disclosed herein, there is no need for the isolation of e.g. white blood cells from whole blood samples. This is of great importance if the assay is used in clinical settings where a large number of samples have to be processed. The methods disclosed herein require smaller tissue samples, preferably blood samples, than the prior art methods.

Methods for detection and/or measurement of polypeptides in biological samples are well known in the art and include, but are not limited to, Western-blotting, Flow cytometry, ELISAs or RIAs, or various proteomics techniques. Monoclonal or polyclonal antibodies recognizing the li p10 peptide or fragments thereof, can either be generated for the purpose of detecting the polypeptides or peptide fragments, e.g. by immunizing rabbits with purified proteins, or known antibodies recognizing the polypeptides or peptide fragments can be used. For example, an antibody capable of binding to the denatured proteins, such as a polyclonal antibody, can be used to detect LI P10 peptide or MHC II polypeptide in a Western Blot. An example for a method to measure a polypeptide is an ELISA. This type of protein quantitation is based on an antibody capable of capturing a specific antigen, and a second antibody capable of detecting the captured antigen. Methods for preparation and use of antibodies, and the assays mentioned hereinbefore are described in Harlow, E. and Lane, D. Antibodies: A Laboratory Manual, (1988), Cold Spring Harbor Laboratory Press.

A preferred method for the detection of li p10 peptide is flow cytometry. Flow cytometry methods are described in Handbook of Flow Cytometry Method, J. Paul Robinson (Editor); Flow Cytometry - A Basic Introduction, Michael G Ormerod (2008) and Current Protocols in Cytometry (2010), Wiley. In this method proteins are not denatured but preserved in their native form.

WO 2010/121918 discloses Cathepsin S inhibitors which can be tested in the methods disclosed herein.

### Short description of the figures

Figure 1: Detection of Ii p10 expression on B cells and T cells using a neoepitope antibody. PBMCs isolated from blood donation of a healthy donor (R213) were incubated for 20h with increasing concentrations of Cathepsin S inhibitor 1 or vehicle. Ii p10 detection on B cells (CD20+) and T cells (CD3+) was determined by flow cytometry using a different neoepitope antibody 7B8 (black).
Figure 2: Detection of Ii p10 expression on B cells using a neoepitope antibody. PBMCs isolated from blood donation of a healthy donor (R172) were incubated for 20 h with increasing concentrations of Cathepsin S inhibitor 1 or vehicle. Ii p10 detection on B cells (CD20+) and T cells (CD3+) was determined by flow cytometry using two different neoepitope antibodies, namely 7B8 (black) and 13C4 (grey). Stain index = Median Fluorescence Intensity (MFI)_{B cells} / MFI_{Tcells}

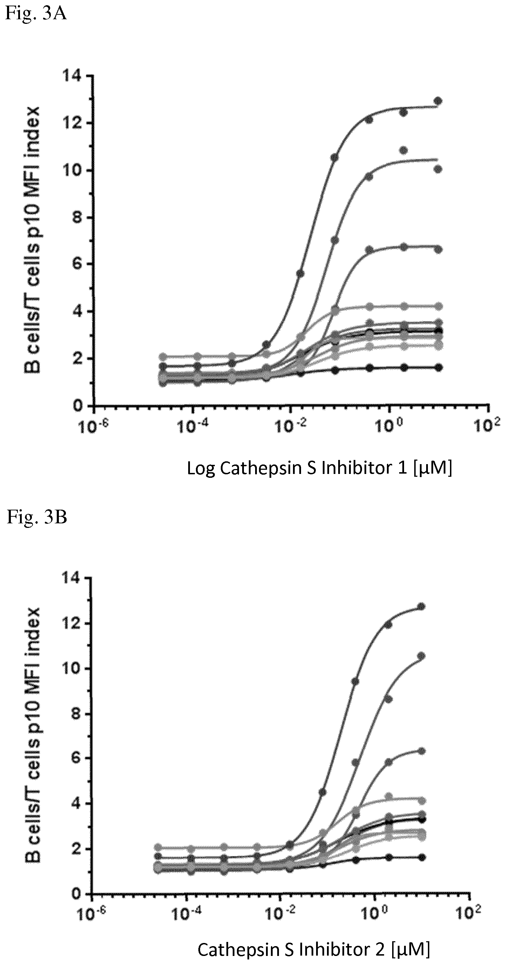

Figure 3 A: Ii p10 accumulates in B cells upon treatment with Cathepsin S inhibitors. PBMCs isolated from blood donations of eleven healthy donors (R39; R185; R198; R204; R209; R140; R154; R175; R213; R4;R214) were incubated for 20h with increasing concentrations of Cathepsin S inhibitor 1 or vehicle. Ii p10 detection on B cells (CD20+) and T cells (CD3+) was determined by flow cytometry using 7B8 neoepitope antibody. Stain index = MFI_{B cells} / MFI_{Tcells}.
Figure 3 B: Ii p10 accumulates in B cells upon treatment with Cathepsin S inhibitors. PBMCs isolated from blood donations of eleven healthy donors (R39; R185; R198; R204; R209; R140; R154; R175; R213; R4;R214) were incubated for 20h with increasing concentrations of Cathepsin S inhibitor 2 or vehicle. Ii p10 detection on B cells (CD20+) and T cells (CD3+) was determined by flow cytometry using 7B8 neoepitope antibody. Stain index = MFI_{B cells} / MFI_{Tcells}.

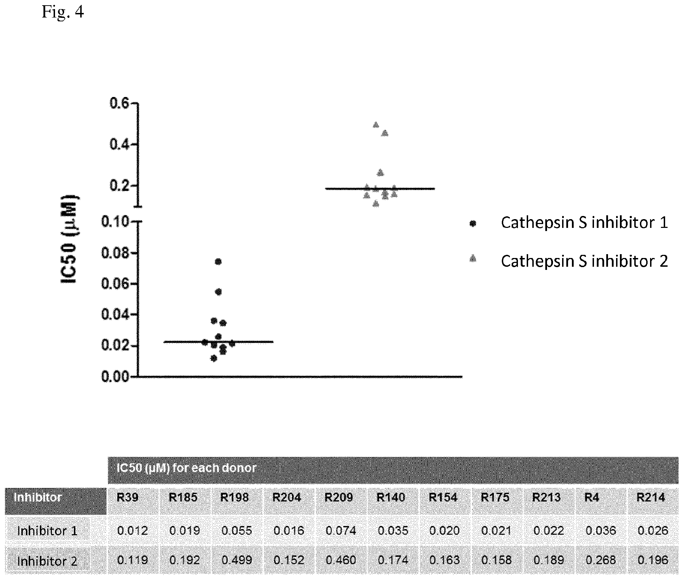

Figure 4: Cathepsin S inhibitor IC50 concentrations. PBMCs isolated from blood donations of eleven healthy donors (R39; R185; R198; R204; R209; R140; R154; R175; R213; R4;R214) were incubated for 20h with increasing concentrations of Cathepsin S inhibitor 1, Cathepsin S inhibitor 2 or vehicle. Ii p10 detection on B cells (CD20+) and T cells (CD3+) was determined by flow cytometry using 7B8 neoepitope antibody. Response curves and IC50 values have been calculated with GraphPad Prism software.

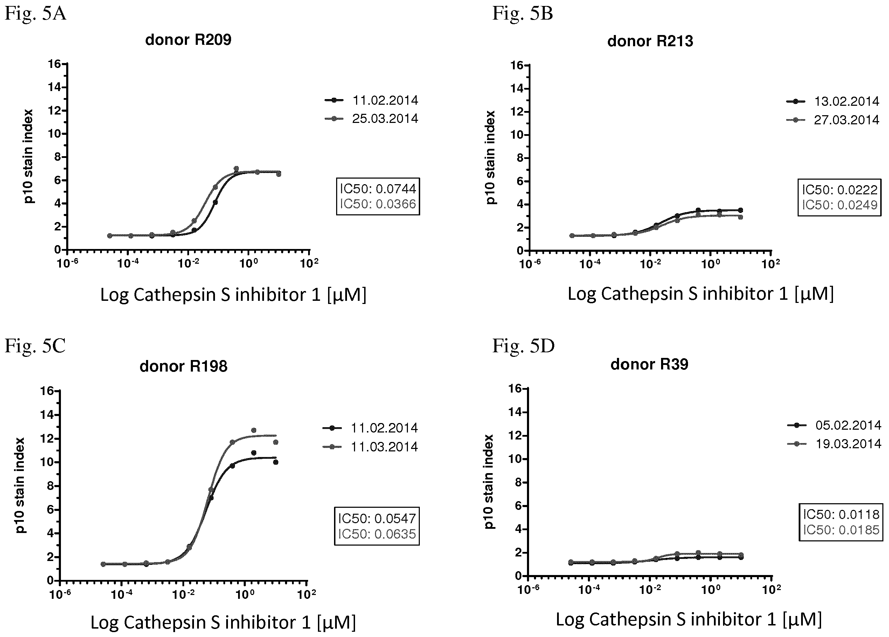

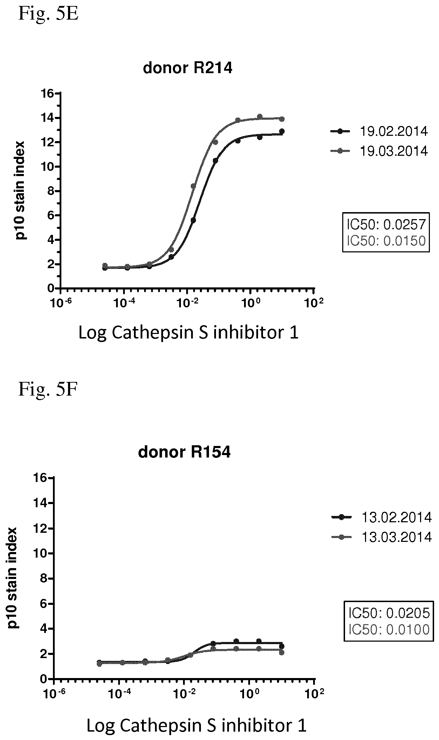

Figures 5 A - F: Ii p10 neoepitope assay longitudinal variability. PBMCs isolated from blood donations of eleven healthy donors (R198; R209; R154; R213; R214) at two time points and were incubated for 20h with increasing concentrations of Cathepsin S inhibitor 1, or vehicle. Ii p10 detection on B cells (CD20+) and T cells (CD3+) was determined by flow cytometry using 7B8 neoepitope antibody.

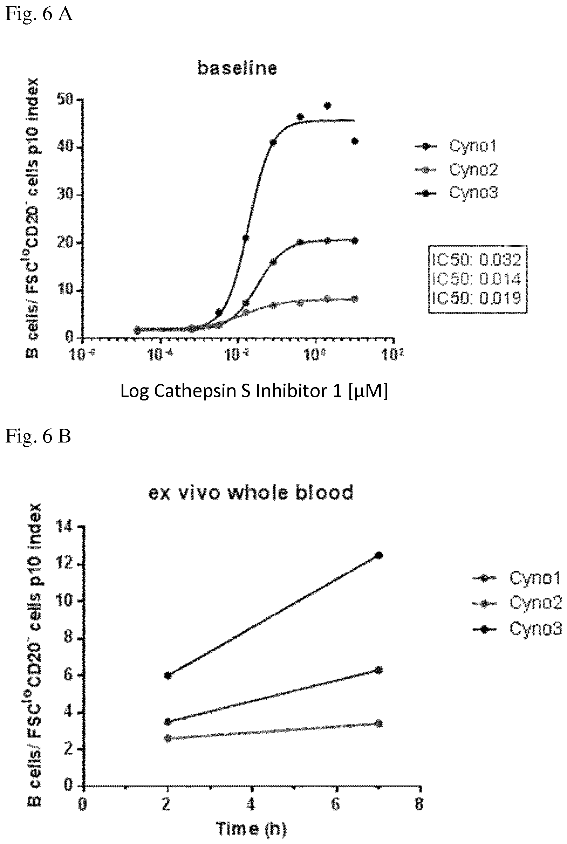

Figure 6 A: Ii p10 accumulation in B cells is detected by the neoepitope assay. Ii p10 detection on B cells (CD20+) was determined by flow cytometry using 7B8 neoepitope antibody clone. PBMCs isolated from 3 cynos (*Macaca fascicularis*) blood donations were incubated for 20h with increasing concentrations of Cathepsin S inhibitor 1 or vehicle. The p10 stain index of *Macaca fascicularis* samples was determined by dividing the Median Fluorescence Intensity (MFI) of B cells by the MFI of Forward Scatter Low CD20 negative (FSCloCD20-) cells.
Figure 6 B: Ii p10 accumulation in B cells is detected by the neoepitope assay after a single oral dose of Cathepsin S inhibitor. Ii p10 detection on B cells (CD20+) was determined by flow cytometry using 7B8 neoepitope antibody clone. Blood was collected from animals 2h and 7h after a single dose of 50mg/kg. Ii p10 neoepitope assay was performed in whole blood. The p10 stain index of *Macaca fascicularis* samples was determined by dividing the Median Fluorescence Intensity (MFI) of B cells by the MFI of Forward Scatter Low CD20 negative (FSCloCD20-) cells.

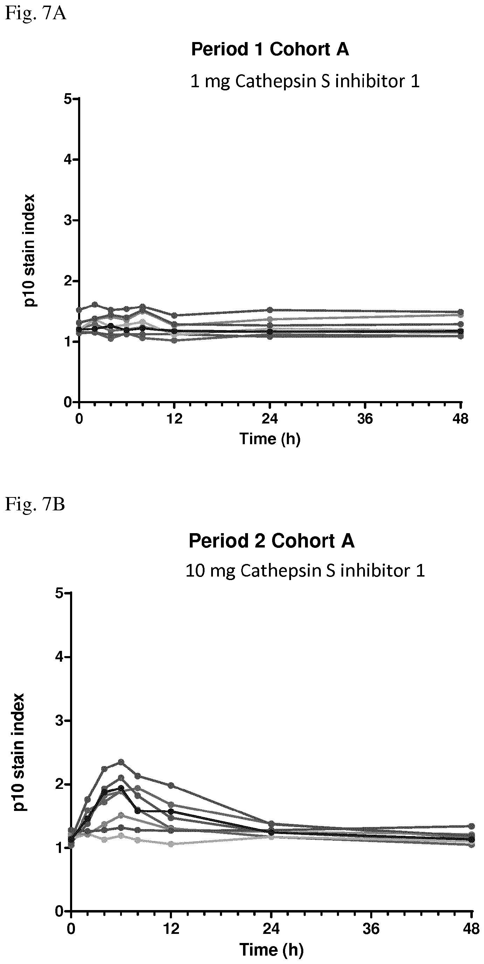

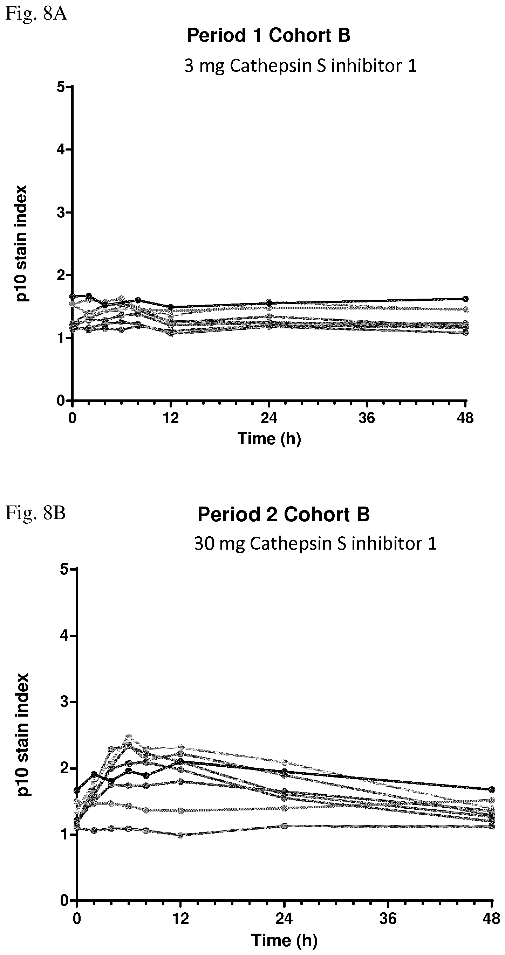

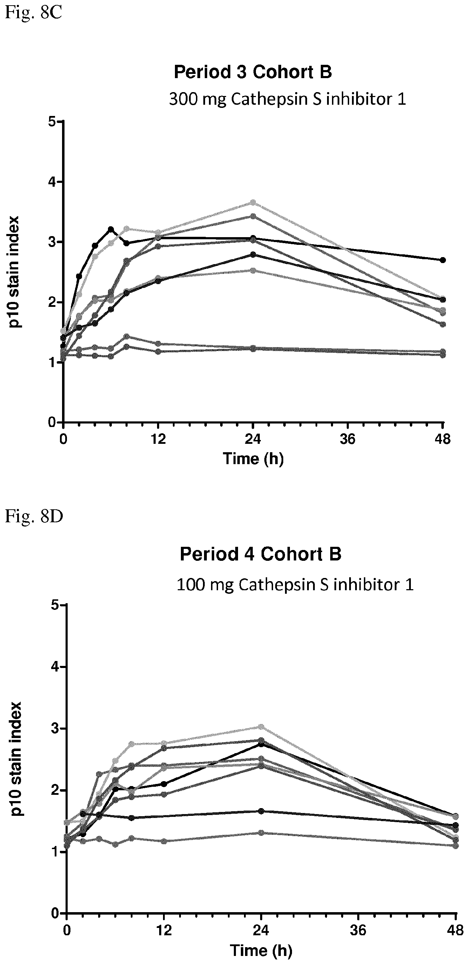

Figure 7A - 7D: Quantification of p10 on samples obtained from subjects participating in a single center, randomized, double-blind, placebo-controlled, single ascending dose study to evaluate the safety, tolerability, pharmacokinetic and pharmacodynamic effects of single doses of Cathepsin inhibitor 1 in healthy male and female volunteers. To account for and limit the impact of the expected inter-subject variability as observed in preclinical experiments, an interleaved cohort ("leapfrog") design was employed. Subjects were recruited into two cohorts of eight subjects each (Cohorts A and B). For each individual within a cohort, the study was a randomized, placebo controlled, four treatment, four periods, four-way crossover. The single dose applied shortly after time point 0 hours is indicated on each figure 7A to7D (cohort A) and figure 8A to 8D (cohort B).

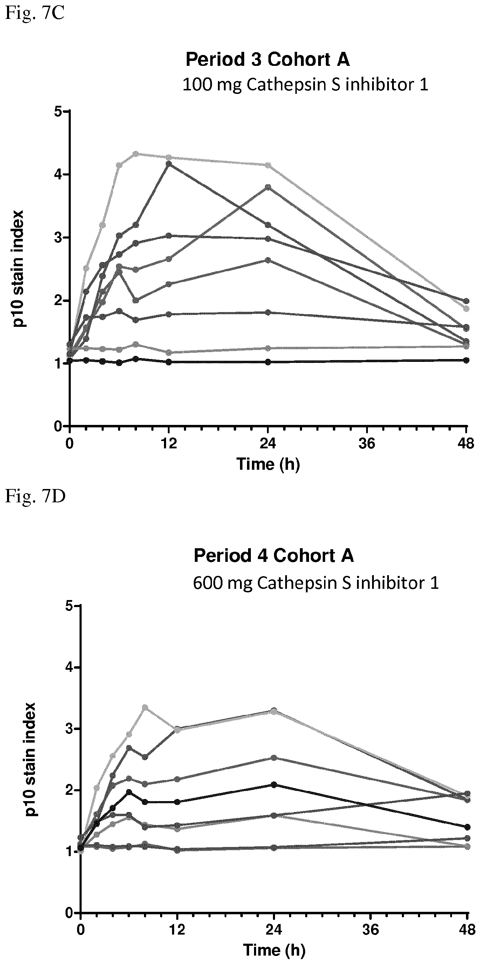

Figure 8A - 8D: Cohort B data, see figure 7 description.

### Experimental part

### In vitro assay with Cathepsin S inhibitor

- Use a sterile Polystyrene 24 well plate
- Add 500µl complete RPMI + Cathepsin S inhibitor with increasing concentrations (0 to 10µM)
- Add 500 µl of PBMC suspension (2x10⁶) to each well
- Mix the content of the wells and incubate the plate for 20h at +37°C, 5% CO2 in a humidified incubator

### FACS staining:

- prepare 1x lyse/fix solution
- add 4mL of pre-warmed 1x lyse/fix solution per 2x10⁶ PBMCs or 200 µl blood
- incubate for 10 min at 37C, wash the cells 1x with PBS (250xg,5minutes, RT), remove supernatant
- add 2mL of 1x permeabilising solution per tube
- incubate for 20 min at RT
- wash the cells 2x with 2ml Cell Stain Buffer (250xg, 5 minutes, RT), remove supernatant
- add 10µg/mL unlabeled anti-p10 antibody in Cell Staining Buffer for 1h at RT
- wash the cells 1x with 2ml Cell Stain Buffer (250xg, 5 minutes, RT), remove supernatant
- add 1:125 PE-conjugated goat anti-rabbit IgG antibody in Cell Staining for 30min at RT in the dark
- wash the cells 2x with 2ml Cell Stain Buffer (250xg, 5 minutes, RT), remove supernatant
- add the antibodies (comp. control) or antibody mixture (diluted in 100µl/sample Cell Stain Buffer) to each tube
- incubate for 20 min at +4°C in the dark
- wash the cells 1x with 2ml Cell Stain Buffer (250xg, 5 minutes, RT), remove supernatant
- resuspend pellet in 200µl Cell Stain Buffer and keep the cells in the dark until the analysis on the FACS

**Antibodies:**

| | | |
|---|---|---|
| goat anti-rabbit IgG PE | Southern Biotech | Cat# 4050-09 |
| | | 1:125/sample |
| CD3 Brilliant Violet 421 | BioLegend | Cat# 300434 |
| | | 5µl/sample |
| CD14 APC | Beckman Coulter | Cat# IM2580 |
| | | 5µl/sample |
| CD20 AlexaFluor 488 | BD Biosciences | Cat# 558056 |
| | | 20µl/sample |
| CD45 PerCP/Cy5.5 | BioLegend | Cat# 304028 |
| | | 2µl/sample |

### Materials and buffers:

24-well plates, BD, order ID: 351147Storage: room temperature, sterile, polystyrene non tissue culture treated

10x BD Phosphoflow Lyse/Fix buffer lysis buffer (BD, order ID: 558049). Storage: +4°C. Dilute the 5x BD Phosphoflow Lyse/Fix buffer with H₂O bidest. to 1x and prewarm it to the required temperature prior to use 10x Phosflow Perm Buffer IV permeabilisation buffer (BD, order ID: 560746). Storage at RT. Dilute 10x BD Phosphoflow Perm Buffer IV with PBS to 1x.

1x Cell Satining Buffer (BioLegend, order ID: 420201. Storage: +4C the 1x Cell Satining Buffer is ready to use. 1x PBS without CaCl₂ and without MgCl₂ (Gibco, order ID: 14190-094). Storage: room temperature. The PBS is ready to use. 5 ml Polystyrene round-bottom tube (FACS tube, BD, order ID: 352052). Storage: room temperature.

**Amino acid sequences**

| **Sequence name** | **Seq. Id. No.** |
|---|---|
| Human Cathepsin S | 1 |
| Human invariant chain li | 2 |
| Human li p10 | 3 |
| VH CDR 1 of anti li p10 antibody 7B8 | 4 |
| VH CDR 2 of anti li p10 antibody 7B8 | 5 |
| VH CDR 3 of anti li p10 antibody 7B8 | 6 |
| VL CDR 1 of anti li p10 antibody 7B8 | 7 |
| VL CDR 2 of anti li p10 antibody 7B8 | 8 |
| VL CDR 3 of anti li p10 antibody 7B8 | 9 |
| VH chain of anti li p10 antibody 7B8 | 10 |
| VL chain of anti li p10 antibody 7B8 | 11 |

## Claims

1. An anti li p10 antibody comprising a VH domain comprising a CDR1 comprising the amino acid sequence of Seq. Id. No. 4, a CDR2 comprising the amino acid sequence of Seq. Id. No. 5, CDR3 sequence comprising the amino acid sequence of Seq. Id. No. 6., and a VL domain comprising a CDR1 comprising the amino acid sequence of Seq. Id. No. 7, a CDR2 comprising the amino acid sequence of Seq. Id. No. 8, a CDR3 sequence comprising the amino acid sequence of Seq. Id. No. 9.

2. The anti li p10 antibody of claim 1, wherein the VH domain comprises the amino acid sequence of Seq. Id. No. 10 and the VL domain comprises the amino acid sequence of Seq. Id. No. 11.

## Patentansprüche

1. Anti li p10 Antikörper, umfassend eine VH-Domäne, umfassend eine CDR1, umfassend die Aminosäuresequenz von SEQ ID NO:4, eine CDR2, umfassend die Aminosäuresequenz von SEQ ID NO:5, eine CDR3-Sequenz, umfassend die Aminosäuresequenz von SEQ ID NO:6, und eine VL-Domäne, umfassend eine CDR1, umfassend die Aminosäuresequenz von SEQ ID NO:7, eine CDR2, umfassend die Aminosäuresequenz von SEQ ID NO:8, eine CDR3-Sequenz, umfassend die Aminosäuresequenz von SEQ ID NO:9.

2. Anti li p10 Antikörper nach Anspruch 1, wobei die VH-Domäne die Aminosäuresequenz von SEQ ID NO:10 umfasst und die VL-Domäne die Aminosäuresequenz von SEQ ID NO:11 umfasst.

## Revendications

1. Anticorps anti li pl0 comprenant un domaine VH comprenant une CDR1 comprenant la séquence d'acides aminés de SEQ ID NO:4, une CDR2 comprenant la séquence d'acides aminés de SEQ ID NO:5, une séquence CDR3 comprenant la séquence d'acides aminés de SEQ ID NO:6, et un domaine VL comprenant une CDR1 comprenant la séquence d'acides aminés de SEQ ID NO : 7, une CDR2 comprenant la séquence d'acides aminés de SEQ ID NO:8, une séquence de CDR3 comprenant la séquence d'acides aminés de SEQ ID NO:9.

2. Anticorps anti li p10 selon la revendication 1, dans lequel le domaine VH comprend la séquence d'acides aminés de SEQ ID NO:10 et le domaine VL comprend la séquence d'acides aminés de SEQ ID NO : 11.


### SEQUENCE LISTING

<110> F. Hoffmann-La Roche AG
<120> Method for monitoring Cathepsin S inhibition
<130> 32272 EP
<150> EP14185409.1
   <151> 2014-09-18
<160> 11
<170> PatentIn version 3.5
<210> 1
   <211> 331
   <212> PRT
   <213> Homo sapiens
<400> 1
<210> 2
   <211> 296
   <212> PRT
   <213> Homo sapiens
<400> 2
   Met His Arg Arg Arg Ser Arg Ser Cys Arg Glu Asp Gln Lys Pro Val
   1               5                   10                  15
   Met Asp Asp Gln Arg Asp Leu Ile Ser Asn Asn Glu Gln Leu Pro Met
               20                  25                  30
   Leu Gly Arg Arg Pro Gly Ala Pro Glu Ser Lys Cys Ser Arg Gly Ala
           35                  40                  45
   Leu Tyr Thr Gly Phe Ser Ile Leu Val Thr Leu Leu Leu Ala Gly Gln
       50                  55                  60
   Ala Thr Thr Ala Tyr Phe Leu Tyr Gln Gln Gln Gly Arg Leu Asp Lys
   65                  70                  75                  80
   Leu Thr Val Thr Ser Gln Asn Leu Gln Leu Glu Asn Leu Arg Met Lys
                   85                  90                  95
   Leu Pro Lys Pro Pro Lys Pro Val Ser Lys Met Arg Met Ala Thr Pro
               100                 105                 110
   Leu Leu Met Gln Ala Leu Pro Met Gly Ala Leu Pro Gln Gly Pro Met
           115                 120                 125
   Gln Asn Ala Thr Lys Tyr Gly Asn Met Thr Glu Asp His Val Met His
       130                 135                 140
   Leu Leu Gln Asn Ala Asp Pro Leu Lys Val Tyr Pro Pro Leu Lys Gly
   145                 150                 155                 160
   Ser Phe Pro Glu Asn Leu Arg His Leu Lys Asn Thr Met Glu Thr Ile
                   165                 170                 175
   Asp Trp Lys Val Phe Glu Ser Trp Met His His Trp Leu Leu Phe Glu
               180                 185                 190
   Met Ser Arg His Ser Leu Glu Gln Lys Pro Thr Asp Ala Pro Pro Lys
           195                 200                 205
   Val Leu Thr Lys Cys Gln Glu Glu Val Ser His Ile Pro Ala Val His
       210                 215                 220
   Pro Gly Ser Phe Arg Pro Lys Cys Asp Glu Asn Gly Asn Tyr Leu Pro
   225                 230                 235                 240
   Leu Gln Cys Tyr Gly Ser Ile Gly Tyr Cys Trp Cys Val Phe Pro Asn
                   245                 250                 255
   Gly Thr Glu Val Pro Asn Thr Arg Ser Arg Gly His His Asn Cys Ser
               260                 265                 270
   Glu Ser Leu Glu Leu Glu Asp Pro Ser Ser Gly Leu Gly Val Thr Lys
           275                 280                 285
   Gln Asp Leu Gly Pro Val Pro Met
       290                 295
<210> 3
   <211> 104
   <212> PRT
   <213> Homo sapiens
<400> 3
   Met His Arg Arg Arg Ser Arg Ser Cys Arg Glu Asp Gln Lys Pro Val
   1               5                   10                  15
   Met Asp Asp Gln Arg Asp Leu Ile Ser Asn Asn Glu Gln Leu Pro Met
               20                  25                  30
   Leu Gly Arg Arg Pro Gly Ala Pro Glu Ser Lys Cys Ser Arg Gly Ala
           35                  40                  45
   Leu Tyr Thr Gly Phe Ser Ile Leu Val Thr Leu Leu Leu Ala Gly Gln
       50                  55                  60
   Ala Thr Thr Ala Tyr Phe Leu Tyr Gln Gln Gln Gly Arg Leu Asp Lys
   65                  70                  75                  80
   Leu Thr Val Thr Ser Gln Asn Leu Gln Leu Glu Asn Leu Arg Met Lys
                   85                  90                  95
   Leu Pro Lys Pro Pro Lys Pro Val
               100
<210> 4
   <211> 10
   <212> PRT
   <213> Oryctolagus cuniculus
<400> 4
   Gly Ile Asp Leu Ser Thr Tyr Ala Met Thr
   1               5                   10
<210> 5
   <211> 14
   <212> PRT
   <213> Oryctolagus cuniculus
<400> 5
   Ile Ile Tyr Asn Asn Gly Val Val Val Tyr Ala Ser Trp Ala
   1               5                   10
<210> 6
   <211> 9
   <212> PRT
   <213> Oryctolagus cuniculus
<400> 6
   Gly Tyr Tyr Asp Gly Ser Val Asn Val
   1               5
<210> 7
   <211> 13
   <212> PRT
   <213> Oryctolagus cuniculus
<400> 7
   Gln Ser Ser Gln Ser Val Tyr Asn Asp Asn Val Leu Ser
   1               5                   10
<210> 8
   <211> 7
   <212> PRT
   <213> Oryctolagus cuniculus
<400> 8
   Leu Ala Ser Thr Leu Ala Ser
   1               5
<210> 9
   <211> 10
   <212> PRT
   <213> Oryctolagus cuniculus
<400> 9
   Leu Gly Gly Tyr Thr Gly Asp Met Tyr Ala
   1               5                   10
<210> 10
   <211> 114
   <212> PRT
   <213> Oryctolagus cuniculus
<400> 10
   Gln Ser Leu Glu Glu Ser Gly Gly Arg Leu Val Thr Pro Gly Thr Pro
   1               5                   10                  15
   Leu Thr Leu Thr Cys Thr Val Ser Gly Ile Asp Leu Ser Thr Tyr Ala
               20                  25                  30
   Met Thr Trp Val Arg Gln Ala Pro Gly Lys Gly Leu Glu Trp Ile Gly
           35                  40                  45
   Ile Ile Tyr Asn Asn Gly Val Val Val Tyr Ala Ser Trp Ala Lys Gly
       50                  55                  60
   Arg Phe Thr Ile Ser Lys Thr Ser Thr Thr Val Asp Leu Lys Met Thr
   65                  70                  75                  80
   Ser Pro Thr Thr Glu Asp Thr Ala Thr Tyr Phe Cys Cys Arg Gly Tyr
                   85                  90                  95
   Tyr Asp Gly Ser Val Asn Val Trp Gly Pro Gly Thr Leu Val Thr Val
               100                 105                 110
   Ser Leu
<210> 11
   <211> 108
   <212> PRT
   <213> Oryctolagus cuniculus
<400> 11
   Gln Val Leu Thr Gln Thr Ala Ser Ser Val Ser Ala Val Val Gly Gly
   1               5                   10                  15
   Thr Val Thr Ile Ser Cys Gln Ser Ser Gln Ser Val Tyr Asn Asp Asn
               20                  25                  30
   Val Leu Ser Trp Phe Gln Gln Lys Pro Gly Gln Pro Pro Lys Leu Leu
           35                  40                  45
   Ile Tyr Leu Ala Ser Thr Leu Ala Ser Gly Val Pro Ser Arg Phe Ser
       50                  55                  60
   Gly Ser Gly Ser Gly Thr Gln Phe Thr Leu Thr Ile Ser Asp Leu Glu
   65                  70                  75                  80
   Cys Asp Asp Ala Ala Ala Tyr Tyr Cys Leu Gly Gly Tyr Thr Gly Asp
                   85                  90                  95
   Met Tyr Ala Phe Gly Gly Gly Thr Glu Val Val Val
               100                 105